# EUROPEAN PATENT APPLICATION

(11) **EP 3 848 023 A1**
(43) Date of publication of application: **14.07.2021**
(21) Application number: 19857203.4
(22) Date of filing: 03.09.2019
(51) Int. Cl.: A61K 31/155, A61P 1/16, A61P 3/00, A61P 3/04, A61P 3/06, A61P 3/10, A61P 5/50, A61P 9/00, A61P 9/10, A61P 9/12, A61P 13/12, A61P 19/06, A61P 43/00

(54) **AMELIORATING AGENT FOR OBESITY-RELATED METABOLIC DISEASE**

(30) Priority: 03.09.2018 JP 2018164819
(71) Applicant: Metabolic and Obese Research Science Corporation, Maebashi-shi, Gunma, 371-0037 (JP)
(72) Inventor: MORI, Masatomo, Maebashi-shi, Gunma 371-0037 (JP)
(74) Representative: Moodie, Samantha Laura
(86) International application number: PCT/JP2019/034675
(87) International publication number: WO 2020/050290

(57) **Abstract**

An object of the present invention is to provide a drug containing, as an active ingredient, a low-molecular-weight compound that ameliorates obesity-related metabolic diseases, for example, exhibits anti-obesity action without reduction of food intake through binding to Helz2 to lower its action, on the basis of the fact that Helz2 is intensely expressed in the liver both in humans and mice, and expression levels are significantly increased in the human liver with NAFLD, as an obesity-related metabolic disease, and in fatty liver in obese mice. The present invention provides an ameliorating agent for an obesity-related metabolic disease, in particular, an ameliorating agent for fatty liver, the ameliorating agent characterized by containing guanabenz or a salt of guanabenz as an active ingredient.

## Description

### Technical Field

The present invention relates to an ameliorating agent for an obesity-related metabolic disease, the ameliorating agent containing, as an active ingredient, a compound that binds to Helz2 (Helicase with zinc finger 2) [also known as peroxisome proliferator-activated receptor (PPAR) DNA-binding domain-interacting protein 1 (PDIP1)], more specifically, an agent containing, as an active ingredient, a compound that ameliorates obesity-related metabolic disease caused by obesity and/or insulin resistance through binding to Helz2 to lower its activity.

### Background Art

### Obesity and Health Problems

As obesity progresses to a higher degree, various health problems arise. In Japan, when a body mass index (BMI) of 25 or higher is presented, the case is defined as obesity. Metabolic disorders induced by obesity cause health problems such as impaired glucose tolerance/diabetes mellitus, dyslipidemia, hypertension, fatty liver, hyperuricemia/gout, coronary artery disease, cerebrovascular disorder, and obesity-related kidney disease, and overweight volumes induced by obesity cause health problems such as sleep apnea, menstrual abnormality, and orthopedic motor system disease. In the case exhibiting over 25 of BMI and one or more health problems among the above-mentioned obesity-relatedhealth problems, or in the case exhibiting a certain area or more of visceral fat in addition to over 25 of BMI, obesity disease is diagnosed (Non Patent Literature 1).

### Insulin Resistance and Obesity-Related Metabolic Disease in Obesity

Metabolic disorders induced by obesity include such obesity-related metabolic diseases that insulin resistance (a state in which insulin does not work well) is strongly involved in the development, such as diabetes mellitus and fatty liver. The hormone that lowers blood glucose in vivo is insulin, which is synthesized and secreted in the pancreatic β cell. A liver is responsible for approximately 90% of gluconeogenesis, and insulin in normal condition suppresses gluconeogenesis in the liver to maintain the constant blood glucose, and also controls fat synthesis in the liver to be constant. During obesity progression, in contrast, most of the cases possess hyperinsulinemia simultaneously with hyperglycemia, not only the development of diabetes mellitus and fatty liver, but also the development of hypertension and hyperlipidemia is promoted (Non Patent Literature 2). Moreover, hyperinsulinemia is also involved in the development of cardiocerebrovascular lesions. In addition, insulin resistance is found in fat and muscle, and involved in the development of obesity-related metabolic disease. In contrast, hepatic insulin resistance occures at an early period in a manner independent of insulin resistance in other organ (Non Patent Literature 3).

### Liver and Insulin Resistance

Insulin resistance in obesity is separately observed between gluconeogenesis system and lipid synthesis system, and selective insulin resistance occurs in a liver (selective hepatic insulin resistance) (Non Patent Literature 4). Consequently, the gluconeogenesis system becomes insulin-resistant, causing hyperglycemia is prevented from being suppressed in spite of hyperinsulinemia. Furthermore, when insulin resistance is preserved for along period of time, pancreatic β cells become disable to maintain high-insulin condition, and the β-cell function gradually becomes exhausted to result in a decrease in insulin secretion, and finally diabetes mellitus is developed.

In contrast, the lipid synthesis system is not vulnerable to insulin-resistant, and hyperinsulinemia promotes lipid synthesis in the liver to lead to the development of non-alcoholic fatty liver disease (NAFLD), which is characterized by excessive accumulation of triglyceride (Tg), and the development of NAFLD further worsen the insulin resistance. Most of the hepatic lipid in NAFLD is derived from enhancement of the lipid synthesis system in the liver, and fatty acid oxidation is reduced in the liver with NAFLD. Therefore, excessive Tg synthesized is not metabolized and Tg is accumulated together with cholesterol in lipid droplet ofhepatocyte. Moreover, hyperinsulinemia condition over along period of time promotes fibrosis and inflammation of the liver with NAFLD together with obesity-related metabolic diseases such as hyperglycemia, hyperlipidemia, and hypertension. Excessively accumulated Tg and cholesterol in the liver act as lipotoxicity to injure hepatocyte, resulting in the development of non-alcoholic steatohepatitis (NASH) (Non Patent Literature 5). Among NAFLD cases untreated for 10 years or longer, 20 to 27% progress into NASH, and hepatic cirrhosis, hepatic failure and liver cancer develop from NASH condition with very high probability.

Therefore, prevention of the development of NASH and amelioration of NASH can be achieved through amelioration of hyperglycemia, hyperlipidemia, and hypertension by reducing body weight and reducing insulin resistance induced by obesity and amelioration of NAFLD by reducing the Tg and cholesterol contents in the liver. Actually, it has been demonstrated that a body weight reduction of 10% reduces fibrosis, a characteristic of NASH (Non Patent Literature 6).

### Cloning of Helz2

By means of a yeast two-hybrid method, the present inventors cloned Helz2 (also known as PDIP 1), as a protein of unknown functions at that time, which binds to the DNA-binding domain of human PPARγ protein, which is a nuclear transcription factor and acts as a master regulator in regulating metabolism (Non Patent Literature 7). Helz2 is a transcriptional coregulator that regulates the gene activity in conjunction with a specific nuclear transcription factor.

### Helz2 Expression and Liver

In mice, Helz2 is the most intensely expressed in hepatocyte among organs that control metabolism, and secondly found in fat (white fat/brown fat) cells in expression levels of 1/2 or lower of those in a liver, but almost no expression is found in muscle or brain hypothalamus. Helz2 exhibits a similar expression pattern in human organs, and is intensely expressed in the liver. Among these organs, the liver is only an organ, in which expression of Helz2 is affected by food intake. Obese mice fed high-fat diet are model animal of human obesity involving hyperphagia, and obesity progressibly involves fatty liver with insulin resistance. As the degree of obesity becomes higher, expression of Helz2 is enhanced in the fatty liver.

### Helz2 Action and Appetite

Obesity occurs as a result of increased appetite and reduction of energy expenditure. Leptin is secreted into blood in proportion to increase in peripheral fat cells, and acts on a brain hypothalamus, a center to control food intake, to reduce appetite. However, human obesity, which involves increased fat cells, shows high blood concentrations of leptin, but food intake is not reduced because of the condition exhibiting central leptin resistance. Likely to the case of human obesity, obese mice fed high-fat diet show the elebated blood concentrations of leptin, but do not show reduction of food intake because of central leptin resistance. Even in Helz2-deficient (knockout) mice, central leptin resistance is sustained, in which food intake is not reduced in spite of the high blood concentrations of leptin, possibly due to almost no expression of Helz2 in the brain hypothalamus (Non Patent Literature 8).

### Helz2 Action and Fatty Liver

In obese mice fed with high-fat diet, hyperinsulinemia and hyperglycemia induced by insulin resistance are found at an early period, and in a long-term course fatty liver similar to NAFLD found in human liver is found in addition to hyperglycemia. In the human liver with NAFLD involving obesity, as with the case of obese mouse fatty liver, Helz2 expression levels clearly increased (Non Patent Literature 8).

In Helz2-deficient mice, in contrast, insulin resistance evoked by obesity was reduced, and hyperinsulinemia and hyperglycemia were mitigated, despite that the mice were grown with high-fat diet. Moreover, Helz2-deficient mice underwent reduction of lipid synthesis in the liver and increases in fatty acid oxidation and enhancement of energy expenditure even with long-term intake of high-fat diet, and as a result Tg content in the liver were decreased, fatty liver was clearly ameliorated, and body weights were also reduced. In brown fat cells and white fat cells of Helz2-deficient mice, no increase of expression of UCP (UCP: uncoupling protein)-1 and UCP-2/3, as markers of energy expenditure due to enhanced fatty acid oxidation, was found, and body temperature increase resulting from the enhanced energy expenditure was not found, either. Further, enhancement of fatty acid oxidation was not found either in muscle of Helz2-deficient mice, and the amount of exercise was not changed. These results indicate that enhancement of energy expenditure is evoked primarily in the peripheral liver in Helz2-deficient condition to cause body weight reduction (Non Patent Literature 8).

In liver of 12-week-old male mice fed methionine- and choline-deficient diet, findings of NASH observed in humans are induced; in Helz2-deficient mice fed the same deficient diet, the marker levels of hepatic disfunction observed in the condition of NASH were clearly decreased and NASH was ameliorated. In non-obese mice fed with low-fat diet, in contrast, deficiency of Helz2 did not affect blood insulin and blood glucose levels (Non Patent Literature 8).

### Helz2 Action and Hepatic Leprb

To search for a molecule that undergoes variation through deficiency of Helz2, cDNA microarray analysis was performed for liver, finding that expression levels of leptin receptor long form (Leprb) were significantly increased in liver of Helz2-deficient mice (Non Patent Literature 8). In contrast, no change was found in Leprb expression in the brain hypothalamus. In obese mice, hepatic Helz2 expression was increased as fatty liver grows, along with decrease in hepatic Leprb expression. Conversely, when Helz2 expression was decreased in fatty liver of obese mice, hepatic Leprb expression was increased (Non Patent Literature 8). Subsequently, it was revealed that AMP-activated protein kinase (AMPK) is localized in downstream signal of hepatic Leprb and phosphorylation thereof is critical for the active action of Leprb. Moreover, it was revealed that an activity of leptin receptor promoter in cultured hepatocyte is promoted on lowering of Helz2 action (lowering of a transcriptional coactivator action) (Non Patent Literature 8).

### Action Mechanism of Helz2

These results clarified that lowering of hepatic Helz2 action changes gene transcriptional regulation followed by activation of leptin receptor promoter to enhance hepatic Leprb expression, subsequently AMPK in the downstream signal is activated to reduce insulin resistance caused by obesity, which ameliorates obesity-related metabolic diseases such as hyperglycemia and fatty liver, and enhances energy expenditure without reduction of appetite, resulting in body weight reduction.

### Amelioration of Insulin Resistance and Clinically Available Drugs

Among oral low-molecular-weight drugs that are currently used in clinical sites and exhibiting hypoglycemic effect, drugs which exhibit ameliorating action on insulin resistance and mitigate hyperglycemia in an insulin-dependent manner, are metformin and pioglitazone (Non Patent Literature 2). A drug that particularly ameliorates NAFLD and NASH among obesity-related metabolic disease based on insulin resistance is pioglitazone, and other drugs have been developed (Non Patent Literature 5). However, a drug that suppresses insulin resistance through binding to Helz2 to lower its action and ameliorates obesity-related metabolic disease has not been provided yet.

### Citation List

### Non Patent Literature

Non Patent Literature 1: Guidelines for the management of obesity disease 2016, Japan Society for the Study of Obesity, Life Science Publishing Co., LTD., 2016.
Non Patent Literature 2: Regulation of hepatic glucose metabolism in health and disease. Nature Rev Endocrinol 13: 572-587, 2017.
Non Patent Literature 3: Hepatokine: linking nonalcoholic fatty liver disorder and insulin resistance, Nature Rev Endocrinol 13: 509-520, 2017.
Non Patent Literature 4: Selective versus total insulin resistance: a pathogenic paradox. Cell Metab 7: 95-96, 2008.
Non Patent Literature 5: Mechanisms of NAFLD development and therapeutic strategies. Nature Medicine 24: 908-922, 2018.
Non Patent Literature 6: Weight loss through lifestyle modification significantly reduces features of nonalcoholic steatohepatitis. Gastroenterology 149: 367-378, 2015.
Non Patent Literature 7: Isolation and characterization of a transcriptional cofactor and its novel isoform that binds the deoxyribonucleic acid-binding domain of peroxisome proliferator-activated receptor-y. Endocrinology 147: 377-388, 2006.
Non Patent Literature 8: Protection against high-fat diet-induce obesity in Helz2-defiecient male mice due to enhanced expression of hepatic leptin receptor. Endocrinology 155: 3459-3472, 2014.

### Summary of Invention

### Technical Problem

The present invention was made in view of the current circumstances as described above, and, on the basis of the fact that Helz2 is intensely expressed in a liver both in humans and mice, and Helz2 expression levels are significantly increased in human liver with NAFLD, as an obesity-related metabolic disease, and in fatty liver in obese mice, an object of the present invention is to provide a drug containing, as an active ingredient, a low-molecular-weight compound that ameliorates obesity-related metabolic disease through binding to Helz2 to lower its action.

### Solution to Problem

Configuration of an ameliorating agent for an obesity-related metabolic disease, which is provided by the present invention to achieve the above object, is characterized by containing guanabenz or a salt of guanabenz as an active ingredient.

Examples of the salt of guanabenz can include an acetate salt of guanabenz.

The obesity-related metabolic disease targeted by the present invention for amelioration is ameliorated through binding of guanabenz or a salt of guanabenz to Helz2 with high affinity to lower its activity.

Examples of the obesity-related metabolic disease include type II diabetes mellitus, fatty liver, dyslipidemia, hypertension, hyperuricemia/gout, coronary artery disease, cerebral infarct, and obesity-related kidney disease, and those caused by obesity and/or insulin resistance are preferred. A drug efficacy of the ameliorating agent of the present invention for an obesity-related metabolic disease is capability of exhibiting anti-obesity action and anti-fatty liver action, in particular, without reduction of appetite. It should be noted that the scope of the fatty liver encompasses non-alcoholic fatty liver disease (NAFLD) and non-alcoholic steatohepatitis (NASH).

The present inventors conducted search for an ameliorating agent for an obesity-related metabolic disease and analysis of pharmacological effect based on achievements of previous studies on the action of Helz2 associated with the development of obesity-related metabolic disease. Specifically, the present inventors selected low-molecular-weight compounds that bind to and dissociate from Helz2 protein with high to middle affinity from 1,200 low-molecular-weight compounds, subsequently identified a low-molecular-weight compound that lowered the Helz2 action by using enhancement of Leprb expression in cultured hepatocyte as an index, and further orally administered the low-molecular-weight compound to obese mice affected by fatty liver involving insulin resistance to analyze ameliorating action on obesity-related metabolic disease, thus, completing the present invention.

### Advantageous Effects of Invention

Through the present invention, an oral low-molecular-weight compound, which binds to Helz2 to lower its action, is selected, and an ameliorating agent for an obesity-related metabolic disease, the ameliorating agent containing this as an active ingredient, was developed. It is preferable that the ameliorating agent of the present invention for an obesity-related metabolic disease be an ameliorating agent that reduces insulin resistance for an obesity-related metabolic disease caused by an obesity and/or insulin resistance.

The ameliorating agent of the present invention for an obesity-related metabolic disease ameliorates type II diabetes mellitus, hypertension, hyperlipidemia, fatty liver, and so on, and, in particular, exhibits anti-obesity action without reduction of appetite, thus being capable of suppressing the development of non-alcoholic fatty liver disease (NAFLD) and non-alcoholic steatohepatitis (NASH) and ameliorating them.

### Brief Description of Drawings

[Figure 1] Figure 1 shows graphs representing results of measurement of intracellular Leprb mRNA through qRT-PCR after addition of guanabenz acetate (Figure 1-A) or salbutamol (Figure 1-B) to the cultured hepatocyte HepG2.
[Figure 2] Figure 2 shows a graph representing temporal variation of body weight in male mouse fed either high-fat diet or low-fat diet.
[Figure 3] Figure 3 shows a graph representing expression of hepatic Leprb mRNA after 14-day oral administration of guanabenz acetate to obese mice fed high-fat diet. In all cases hereinafter, black columns each represent a group with administration of guanabenz acetate.
[Figure 4] Figure 4 shows graphs representing temporal variation of body weight gain (Figure 4-A) and body weight (Figure 4-B) with oral administration of guanabenz acetate to obese mice, where the data begin from pre-administration.
[Figure 5] Figure 5 shows graphs representing blood leptin concentrations (Figure 5-A) after 14-day oral administration of guanabenz acetate to obese mice, and temporal variation of daily intake (Figure 5-B) and total food intake accumulation (Figure 5-C).
[Figure 6] Figure 6 shows graphs representing the HOMA-IR index (Figure 6-A), an insulin resistance index, blood insulin levels (Figure 6-B), and blood glucose levels (Figure 6-C) after 14-day oral administration of guanabenz acetate to obese mice.
[Figure 7] Figure 7 shows graphs representing pancreatic weight (Figure 7-A) and the HOMA-β index (Figure 7-B), an index of pancreatic β-cell function, after 14-day oral administration of guanabenz acetate to obese mice.
[Figure 8-1] Figure 8-1 shows graphs representing hepatic triglyceride content (Tg, Figure 8-A) and cholesterol content (Chol, Figure 8-B) after 14-day oral administration of guanabenz acetate to obese mice.
[Figure 8-2] Figure 8-2 shows amounts of fat accumulated in hepatocyte per cell and change in shape of fat. As shown in representative photographs, fat globules in hepatocytes positive in BODIPY staining are exhibited (Figure 8-C). A graph representing area of staining-positive cell (Figure 8-D) and a graph representing size of staining-positive cell (Figure 8-E) for different groups are shown. "Administration of G agent" refers to administration of guanabenz acetate.
[Figure 9] Figure 9 shows graphs representing weight change of liver (Figure 9-A) and heart (Figure 9-B) after 14-day oral administration of guanabenz acetate to obese mice.
[Figure 10] Figure 10 shows graphs representing blood chylomicron-triglyceride (CM-Tg, Figure 10-A), very-low-density lipoprotein-triglyceride (VLD-Tg, Figure 10-B), low-density lipoprotein-cholesterol (LDLc, Figure 10-C), and high-density lipoprotein-cholesterol (HDLc,
Figure 10-D) levels after 14-day oral administration of guanabenz acetate to obese mice.
[Figure 11] Figure 11 shows graphs representing weight change s in spleen (Figure 11-A) and kidney (Figure 11-B) after 14-day oral administration of guanabenz acetate to obese mice.
[Figure 12] Figure 12 shows graphs representing weights of intestinal membrane fat (Figure 12-A), peritesticular fat (Figure 12-B), total visceral fat (Figure 12-C), soleus muscle (Figure 12-D), plantaris muscle (Figure 12-E), and gastrocnemius muscle (Figure 12-F) after 14-day oral administration of guanabenz acetate to obese mice.

### Description of Embodiments

Guanabenz, which is an active ingredient of an ameliorating agent of the present invention for metabolic control disorder, is a known compound that is represented by a formula: 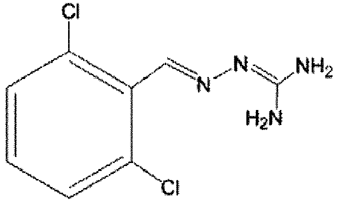 , acts as a central α2-adrenaline receptor agonist, attenuates a sympathetic nervous action for heart to reduce blood pressure, and is used in clinical site as a therapeutic drug for hypertension to ameliorate peripheral vascular resistance. The Ki value of guanabenz to α2-adrenaline receptor is 7 × 10⁻⁹ M.

The molecular formula of guanabenz is C₈H₈Cl₂N₄ and the molecular weight is 291.1, and the name defined by International Union of Pure and Applied Chemistry is 2-{[(2,6-dichlorophenyl)methylidene]amino}guanidine. The half-life of guanabenz in blood is 4.3 to 6.4 hours, and an absorptivity from a gastrointestinal tract is approximately 75%, and guanabenz is mostly metabolized in the liver.

The ameliorating agent of the present invention for an obesity-related metabolic disease contains guanabenz or a salt thereof such as acetate salt thereof as an active ingredient, and is formulated into an appropriate dosage form, singly or together with a pharmaceutically acceptable additional component.

The ameliorating agent of the present invention for an obesity-related metabolic disease may be in a dosage form to be used as a pharmaceutical composition, and can be formulated, for example, as any of tablet (including orally disintegrating tablet), capsule, controlled - release formulation, enteric-coated formulation, granule, powder, syrup, troche, rectal formulation, injection, or transdermal formulation, by using a known method. In manufacturing a formulation, formulation may be performed in accordance with regulation and rule in any country, and can be performed in accordance with the latest Japanese pharmacop oeia, for example, The Japanese Pharmacopoeia 17th Edition.

Excipient to be contained in such pharmaceutical formulation is not limited, and in an example the following can be appropriately combined for use: diluents such as starch, mannitol, and lactose; binders such as carboxymethyl cellulose sodium and hydroxypropyl cellulose; disintegrators such as microcrystalline cellulose and carboxymethyl cellulose; lubricants such as talc and magnesium stearate; and flow improvers such as light anhydrous silicic acid.

A liquid such as an injection can be employed as a pharmaceutical formulation, and, for example, a formulation obtained by dispersing or solubilizing in physiological saline, distilled water, or the like containing a surfactant, a dispersant, or the like may be employed, and a lyophilized formulation that is dispersed or solubilized at time of use, as necessary, may be employed. A pH adjustor or stabilizer may be added to a liquid.

A method of administration of an ameliorating agent of the present invention for an obesity-related metabolic disease can be appropriately set in a manner compatible with symptom of an obesity-related metabolic disease of interest or a patient therewith.

A dose within the range of 0.01 to 1000 mg may be typically administered per day, and the dose may be 0.1 mg or more, 1 mg or more, or 500 mg or less, 200 mg or less, 100 mg or less, 50 mg or less, or 20 mg or less within that range.

The frequency of administration is not limited, and may be one administration per day or multiple administrations per day. Administration may be performed at intervals of 1 day or longer. The timing of administration is appropriately set, for example, before meal, after meal, or during meal.

Even the route of administration is not limited, and administration can be performed with a dosage form of any of oral agent, nasal drop, oro - mucosal agent, vaginal agent, suppository, injection, and so on, and oral administration or parenteral administration is performed, and examples of parenteral administration include intravenous, intraarterial, intramuscular, subcutaneous, intratissue, intranasal, intradermal, intracerebral, intrarectal, intravaginal, intraperitoneal administrations, and so on. In being administered as an injection, the injection may be administered in one shot or through infusion.

There is no limitation to patient for administration, but administration to human is preferred.

The present invention may include a method for ameliorating an obesity-related metabolic disease by administrating a therapeutically effective amount of guanabenz or a salt of guanabenz to a patient in need thereof.

In the present invention, an ameliorating agent containing guanabenz or a salt of guanabenz for an obesity-related metabolic disease can be administered to a patient in need thereof.

The ameliorating agent for an obesity-related metabolic disease can be a prophylactic or therapeutic agent for an obesity-related metabolic disease, and the method for ameliorating an obesity-related metabolic disease can be a prophylactic or therapeutic method for an obesity-related metabolic disease.

The present invention also provides the following.

Guanabenz or a salt of guanabenz for ameliorating an obesity-related metabolic disease.

Guanabenz or a salt of guanabenz for preventing or treating an obesity-related metabolic disease.

Use of guanabenz or a salt of guanabenz for ameliorating an obesity-related metabolic disease.

Use of guanabenz or a salt of guanabenz for preventing or treating an obesity-related metabolic disease.

Use of guanabenz or a salt of guanabenz for adjustment/manufacture of a pharmaceutical composition/pharmaceutical formulation for ameliorating an obesity-related metabolic disease.

Use of guanabenz or a salt of guanabenz for adjustment/manufacture of a pharmaceutical composition/pharmaceutical formulation for preventing or treating an obesity-related metabolic disease.

The obesity-related metabolic disease in each embodiment described herein may be an obesity-related metabolic disease caused by insulin resistance and/or an obesity-related metabolic disease caused by obesity. Examples of the obesity-related metabolic disease in the present invention include, but are not limited to, impaired glucose tolerance or diabetes mellitus, dyslipidemia, hypertension, hyperuricemia or gout, coronary artery disease, cerebral infarct, fatty liver, and obesity - related kidney disease. Especially, fatty liver is preferred as the obesity-related metabolic disease, and the obesity-related metabolic disease may be non-alcoholic fatty liver disease or non-alcoholic steatohepatitis. In the present invention, the obesity-related metabolic disease caused by insulin resistance and/or caused by obesity may be fatty liver or fatty liver caused by insulin resistance and/or caused by obesity, and such fatty liver may be non-alcoholic fatty liver disease or non-alcoholic steatohepatitis.

The ameliorating agent for various obesity-related metabolic diseases may exhibit anti-obesity action without reduction of appetite, and may be an ameliorating agent for obesity-related metabolic disease including metabolic syndrome.

In the present invention, the salt of guanabenz to be used for ameliorating or preventing or treating an obesity-related metabolic disease is not limited, but preferably guanabenz acetate.

An ameliorating agent of the present invention for an obesity-related metabolic disease may be used in combination with an additional drug known to be used for obesity-related metabolic disease, and may be formulated into a drug combination. In use in combination, any of a kit formulation, independent pharmaceutical formulation, and a drug combination is acceptable.

The additional drug known to be used for obesity-related metabolic disease is not limited, and any drug known to be applicable for preventing or treating impaired glucose tolerance or diabetes mellitus, dyslipidemia, hypertension, hyperuricemia or gout, coronary artery disease, cerebral infarct, fatty liver (including non-alcoholic fatty liver disease and non-alcoholic steatohepatitis), or obesity-related kidney disease may be used.

Examples of mode of administration include, but are not limited to:
oral administration of a single agent of guanabenz or an acetate salt thereof as any of tablet (including orally disintegrating tablet), capsule, controlled-release formulation, enteric-coatedformulation, granule, powder, syrup, or troche;
parenteral administration of guanabenz or an acetate salt thereof as any of rectal formulation, injection, or transdermal formulation;
administration of a combination drug of guanabenz or an acetate salt thereof and a metformin agent in any of the above mode of administration;
administration of a combination drug of guanabenz or an acetate salt thereof and a pioglitazone agent in any of the above mode of administration;
administration of a combination drug of guanabenz or an acetate salt thereof and a dipeptidyl peptidase-4 inhibitor in any of the above mode of administration;
administration of a combination drug of guanabenz or an acetate salt thereof and an α-glucosidase inhibitor in any of the above mode of administration;
administration of a combination drug of guanabenz or an acetate salt thereof and a sodium/glucose cotransporter-2 inhibitor in any of the above mode of administration;
administration of a combination drug of guanabenz or an acetate salt thereof and a sulfonylurea-based drug in any of the above mode of administration;
administration of a combination drug of guanabenz or an acetate salt thereof and a glinide-based drug in any of the above mode of administration; and
administration of a combination drug of guanabenz or an acetate salt thereof and a glucagon-like peptide-1 analog or a receptor agonist therefor in any of the above mode of administration.

### Examples

### Procedures

### 1. High-Throughput Screening for Low-Molecular-Weight Compounds That Bind to Helz2

HEK293 cells were allowed to express Helz2 by using a Helz2 expression vector (p3xFLAG-CMV10-pDIP2), and Helz2 protein was purified. Subsequently, high-throughput screening assay through surface plasmon resonance (Flexera Japan KK, Tokyo) was performed for low-molecular-weight compounds that bind to Helz2 using an array panel holding 1,200 low-molecular-weight compounds, all approved by the U.S. Food and Drug Administration, to find low-molecular-weight compounds that showed binding and dissociation against Helz2.

### 2. Identification of Low-Molecular-Weight Compound That Promotes Leprb Expression in Cultured Hepatocytes

Because Leprb gene expression is promoted on lowering of the action of Helz2 through binding of a low-molecular-weight compound, the low-molecular-weight compounds that bound to Helz2 were screened with use of increase of Leprb gene expression in cultured hepatocyte as an index. Each of the low-molecular-weight compounds that bound to and dissociated from Helz2 with high to middle affinity (Sigma-Aldrich Co. LLC) was diluted to a final concentration of 10⁻⁹ to 10⁻⁷ M, and added to HepG2 cells, a cultured hepatocyte strain (10⁵ cells/well in a 6-well plate). The hepatocytes were cultured at 37°C for 24 to 48 hours, and Leprb gene expression therein was measured through a quantitative real time polymerase chain reaction (qRT-PCR, TaqMan Gene expression assays using LEPRB probes) method.

### 3. Hepatic Leprb Expression with Oral Administration of Low-Molecular-Weight Compound

Six-week-old male mice (C57BL6J) were grown with high-fat diet (fat content: 60 kcal%, Research diets, Inc.) or low-fat diet (fat content: 10.5 kcal%, CLEA Japan, Inc.) for 18 weeks. Thereafter, obese mice fed high-fat diet were separated into two groups, and 0.3 mL of physiological saline was orally administered to one group through a gastric tube for mouse, and 0.3 mL of a solution of a low-molecular-weight compound dissolved in physiological saline was orally administered to the other group through the gastric tube for mouse. Oral administration was performed every day over 14 days during 30 minutes before turning lights off at 18:00 (turning lights on at 6:00, 12-hour light-dark cycle, room temperature: 22°C). On day 15, fasting was initiated at 9:00, each mouse was anesthetized with isoflurane at 14:00, blood was collected from the abdominal inferior vena cava (blood glucose was measured for tail vein blood), and the weight of each organ was measured. Thereafter, the organs were stored at -80°C. Hepatic Leprb expression was measured by means of the qRT-PCR.

### 4. Daily Variation of Body Weight with Oral Administration of Low-Molecular-Weight Compound

The body weight of each mouse with administration of physiological saline or administration of a low-molecular-weight compound was measured every day before each administration at 18:00.

### 5. Change in Blood Leptin Concentration and Food Intake after Oral Administration of Low-Molecular-Weight Compound

Blood leptin concentration after administration of physiological saline or administration of a low-molecular-weight compound were measured by using a mouse leptin ELISA kit (Morinaga Institute of Biological Science, Inc.). The daily food intake of each mouse was measured every day before each administration at 18:00.

### 6. Change in Insulin Resistance and Blood Glucose Level after Oral Administration of Low-Molecular-Weight Compound

Fasting blood glucose and blood insulin concentration after administration of physiological saline or administration of a low-molecular-weight compound were measured respectively by using an enzyme-electrode method with glucose oxidase and a mouse insulin ELISA kit (Morinaga Institute of Biological Science, Inc.). The insulin resistance of each mouse was represented as Homeostatic model assessment of insulin resistance (HOMA-IR) [insulin (mU/L) × blood glucose (mM) 22.5] index (×100) (Brit J Nutr 101: 701-708, 2009).

7. Change in Pancreatic Weight and Pancreatic β-Cell Function after Oral Administration of Low-Molecular-Weight Compound

Pancreatic weight after administration of physiological saline or administration of a low-molecular-weight compound was measured. The pancreatic β-cell function of each mouse was represented by Homeostatic model assessment-β cell function (HOMA-β) {[insulin (mU/L) × 20] ÷ [blood glucose (mM) - 3.5]} index (×100) (Brit J Nutr 101:701-708, 2009).

### 8. Change in Hepatic Lipid Content after Oral Administration of Low-Molecular-Weight Compound

Hepatic Tg and Chol contents after administration of physiological saline or administration of a low-molecular-weight compound were measured by using a LipoSEARCH method (Skylight Biotech, Inc.).

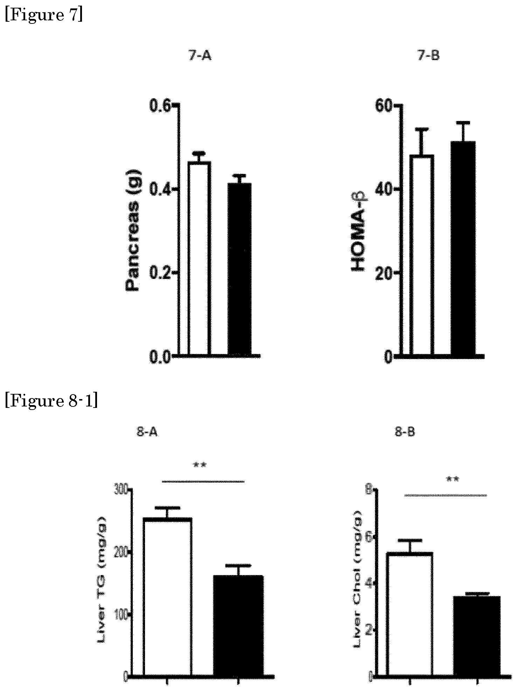

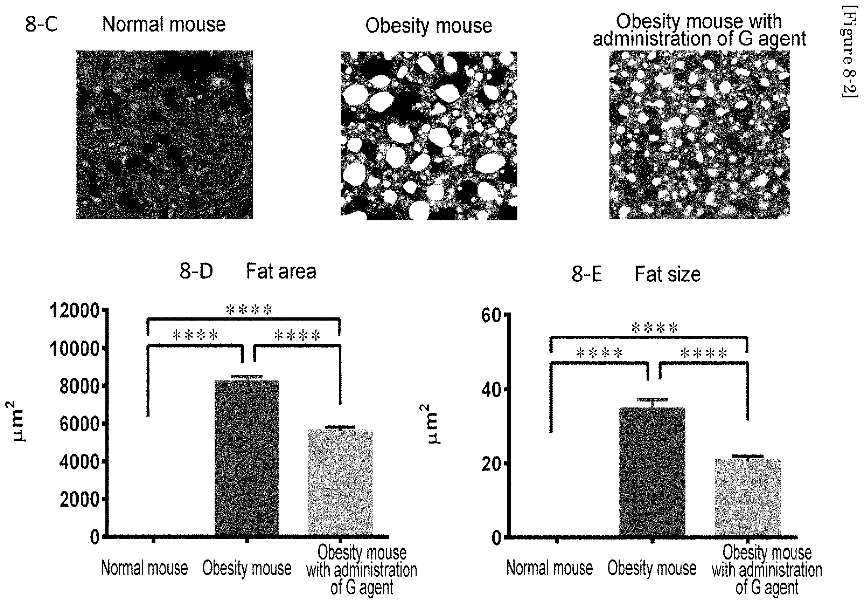

To visualize and quantify the amount of fat accumulated in hepatocyte per cell and change in shape of fat, fat globule fluorescent staining with BODIPY (excitation light: 493 nm; fluorescence: 503 nm) was performed. Staining-positive area and size of staining-positive cell were measured in 10 views per mouse hepatocyte in each group. ANOVA was used for intergroup significance test in Figures 8-D and 8-E (****. P < 0.001).

9. Change in Weights of Liver and Heart after Oral Administration of Low-Molecular-Weight Compound

The weights of the liver and heart after administration of physiological saline or administration of a low-molecular-weight compound were measured.

### 10. Change in Blood Lipid Concentration after Oral Administration of Low-Molecular-Weight Compound

Fasting blood CM-Tg, VLDL-Tg, LDLc, and HDLc concentrations after administration of physiological saline or administration of a low-molecular-weight compound were measured by using the LipoSEARCH method.

### 11. Change in Weights of Spleen and Kidney after Oral Administration of Low-Molecular-Weight Compound

The weights of the spleen and kidney after administration of physiological saline or administration of a low-molecular-weight compound were measured.

### 12. Change in Weights of Fat and Muscle after Oral Administration of Low-Molecular-Weight Compound

The amounts of visceral fat, which evokes metabolic disorder, and muscle (right side) after administration of physiological saline or administration of a low-molecular-weight compound were measured.

### 13. Significance Test

Data were represented as mean ± standard deviation for each group. Paired Student's t test was used for significance test among two groups, and two-way ANOVA followed by Bonferroni's test was used for significance test for three or more, multiple groups.

### Results

### 1. High-Throughput Screening for Low-Molecular-Weight Compounds That Bind to Helz2

Analysis was performed for 1,200 low-molecular-weight compounds that bind to and dissociate from Helz2. Selected considering the result were 14 low-molecular-weight compounds that bound and dissociated with high to middle affinity (KD value: 10⁻⁹ to 4.9 ×10⁻⁷ M) (amifostine trihydrate, brompheniramine maleate, clemastine fumarate, fenbendazole, guanabenz acetate, loperamide hydrochloride, pirenperone, ritodrine hydrochloride, rivastigmine tartrate, salbutamol, sibutramine hydrochloride, sulbactam, voriconazole, zoxazolamine). Other low-molecular-weight compounds exhibited low affinity with KD values of 5 × 10⁻⁷ M or higher.

### 2. Identification of Low-Molecular-Weight Compound Guanabenz That Promotes Leprb Expression in Cultured Hepatocyte

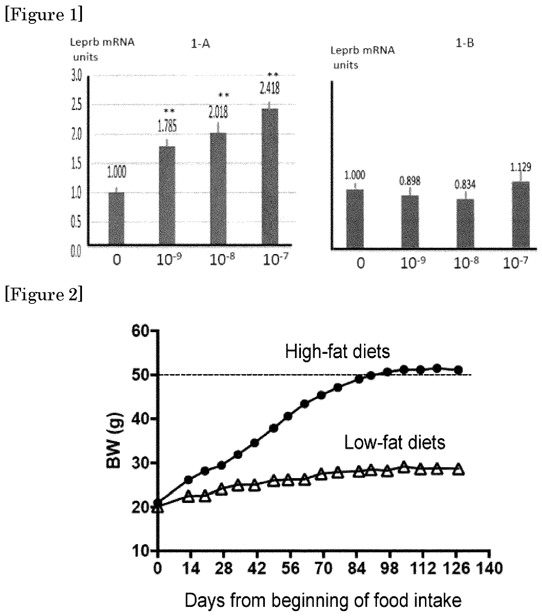

Concentration-dependent increase in Leprb expression by each of the 14 low-molecular-weight compounds added to cultured hepatocyte was used as an index of lowering of Helz2 action, and guanabenz acetate, a candidate low-molecular-weight compound, was identified (Figure 1-A). The KD value for binding to and dissociating from Helz2 was 2.3 × 10⁻⁹ M, indicating high affinity. In contrast, addition of salbutamol, which exhibited middle affinity with Helz2 as 2.2 × 10⁻⁷ M, caused no significant influence on Leprb expression (Figure 1-B). The KD values of clonidine hydrochloride and methyldopa, each of which acts as a central α2-adrenaline receptor agonist to exhibit hypotensive action similarly to guanabenz, for Helz2 were 10⁻⁶ M or higher, indicating low affinity.

### 3. Hepatic Leprb Expression after Oral Administration of Guanabenz

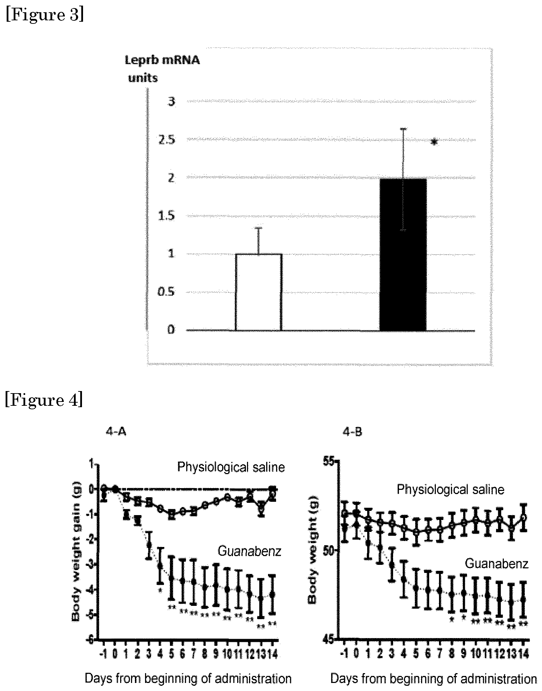

The body weight of male mice reached a plateau of approximately 50 g at 14 weeks after the beginning of intake of high-fat diet (N = 16), indicating significant obesity as compared with male mice with intake of low-fat diet (N = 4) (Figure 2). The dose of guanabenz acetate orally administered was set to a 0.32 mg/kg body weight of mouse/day, a usual dose for humans considering the half-life of guanabenz acetate in blood, the absorptivity from the gastrointestinal tract, and so on. Hepatic Leprb gene expression was significantly increased after 14-day continuous oral administration of guanabenz acetate to obese mice fed high-fat diet for 18 weeks (black column, N = 8), as compared with that after administration of physiological saline (white column, N = 8) (Figure 3).

### 4.Daily Variation of Body Weight with Oral Administration of Guanabenz

No body weight change was found with administration of physiological saline to obese mice (white circles, N = 8) (Figure 4). In contrast, the body weight gain was significantly reduced with administration of guanabenz acetate (black circles, N = 8) after day 4 from the beginning of administration (Figure 4-A), and significant body weight reduction was found on day 8 from the beginning of administration (Figure 4-B), and this body weight reduction was maintained during the period of administration. These results indicate that anti-obesity action is found with administration of guanabenz acetate even in a state with high-fat diet.

### 5. Change in Blood Leptin Concentration and Food Intake after Oral Administration of Guanabenz

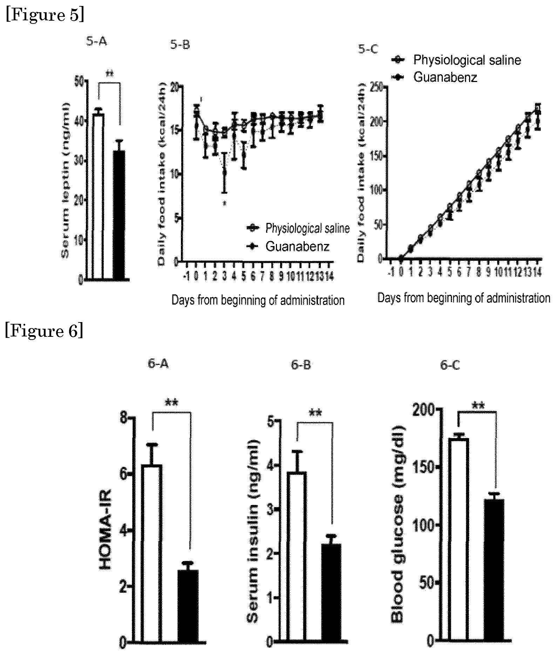

The increased blood leptin concentration found in obese mice was rather slightly decreased after administration of guanabenz acetate (black column, N = 8) as compared with that after administration of physiological saline (white column, N = 8), and the concentration was 10-fold or more higher than the leptin concentration in non-obese mice (2.3 ± 0.5 ng/mL, N = 4) (Figure 5-A). After administration of guanabenz acetate to obese mice (black circles, N = 8), no change in daily food intake was found except on day 3 from the beginning of administration, as compared with that after administration of physiological saline (white circles, N = 8) (Figure 5-B), and no significant change was found in total food intake accumulation (Figure 5-C), similarly. These results revealed that oral administration of guanabenz acetate in a usual dose does not reduce appetite even under growing with high-fat diet, and provides anti-obesity action through energy expenditure enhancing action.

### 6. Change in Insulin Resistance and Blood Glucose Level after Oral Administration of Guanabenz

The HOMA-IR index (Figure 6-A), which is an insulin resistance index, blood insulin levels (Figure 6-B), and blood glucose levels (Figure 6-C) in obese mice fed high-fat diet were higher than the indexes in non-obese mice (HOMA-IR index: 0.65 ± 0.12, insulin level: 0.48 ± 0.08 ng/mL, blood glucose level: 142.8 ± 9.8 mg/dL, N = 4). Administration of guanabenz acetate to obese mice (black column, N = 8) suppressed the HOMA-IR index, mitigated hyperinsulinemia, and lowered the level of hyperglycemia to that in non-obese mice, as compared with administration of physiological saline (white column, N = 8). These results indicate that 2-week administration of guanabenz significantly reduces insulin resistance to ameliorate hyperglycemia as an obesity-related metabolic disease.

### 7. Change in Pancreatic Weight and Pancreatic β-Cell Function after Oral Administration of Guanabenz

The pancreatic weight of obese mice was more than the pancreatic weight of non-obese mice (0.34 ± 0.01 g, N = 4), and the pancreatic weight increased by obesity was not changed after administration of guanabenz acetate (black column, N = 8) as compared with that after administration of physiological saline (white column, N = 8) (Figure 7-A). The HOMA-β index, an index of pancreatic β-cell function, was not changed after administration of guanabenz acetate, similarly (Figure 7-B). These results indicate that administration of guanabenz in a usual dose causes no influence on pancreatic β-cell function.

### 8. Change in Hepatic Lipid Content after Oral Administration of Guanabenz

The hepatic Tg content and Chol content in fatty liver (NAFLD) found in obese mice fed high-fat diet were significantly higher than those in liver of non-obese mice (Tg content: 11.3 ± 2.6 mg/g, Chol content: 2.5 ± 0.1 mg/g, N = 4). The increased hepatic Tg content (Figure 8-A) and Chol content (Figure 8-B) were significantly reduced after administration of guanabenz acetate (black column, N = 8), as compared with those after administration of physiological saline (white column, N = 8). These results indicate that even 2-week oral administration of guanabenz ameliorates fatty liver characterized by Tg and Chol accumulations, as an obesity-related metabolic disease.

In addition, the fat globule area and fat globule cell size, which reflect the amount of fat per hepatocyte, in fatty liver caused by intake of high-fat diet significantly decreased after administration of guanabenz acetate (Figures 8-C to 8-E), which indicated anti-fatty liver action of guanabenz acetate.

### 9. Change in Weights of Liver and Heart after Oral Administration of Guanabenz

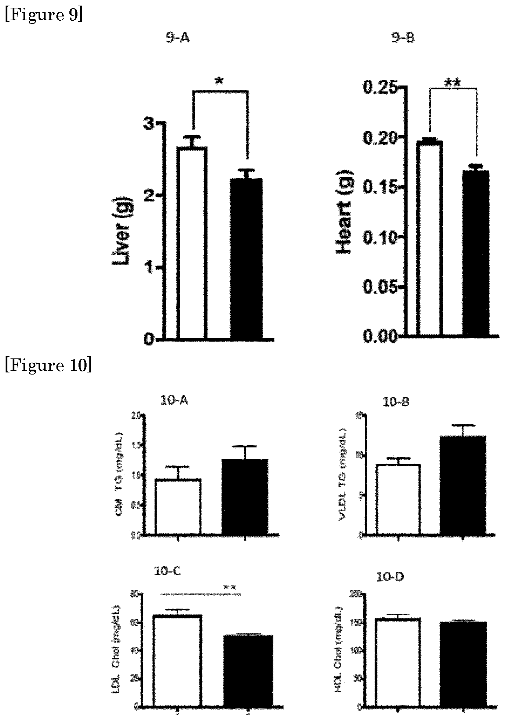

The weights of liver (Figure 9-A) and heart (Figure 9-B) of obese mice increased over those of non-obese mice (liver: 1.17 ± 0.02 g, heart: 0.14 ± 0.09 g, N = 4), and the weights were significantly decreased after administration of guanabenz acetate (black column, N = 8) as compared with those after administration of physiological saline (white column, N = 8). These results indicate that liver enlargement evoked by obesity is reduced in association with amelioration of fatty liver with administration of guanabenz acetate. Further, it is suggested that guanabenz acetate mitigates heart burden induced by obesity through central α2-adrenaline receptor action.

### 10. Change in Blood Lipid Concentration after Oral Administration of Guanabenz

The blood CM-Tg (Figure 10-A), VLDL-Tg (Figure 10-B), and HDLc (Figure 10-D) concentrations in obese mice were not changed after administration of guanabenz acetate (black column, N = 8) as compared with those after administration of physiological saline (white column, N = 8), but the LDLc concentration (Figure 10-C), which acts to cause promotion of arteriosclerosis, was significantly decreased after administration of guanabenz acetate.

### 11. Change in Weights of Spleen and Kidney after Oral Administration of Guanabenz

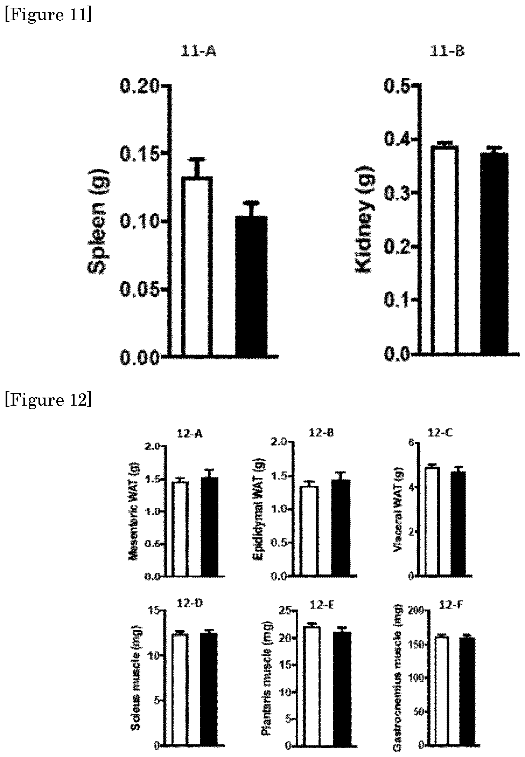

No change was found in the weights of spleen and kidney after administration of guanabenz acetate (black column, N = 8) as compared with those after administration of physiological saline (white column, N = 8) (Figures 11-A, 11-B).

### 12. Change in Weights of Fat and Muscle after Oral Administration of Guanabenz

The amounts of intestinal membrane fat (Figure 12-A), peritesticular fat (Figure 12-B), and total visceral fat (Figure 12-C) increased with obesity were not changed after administration of guanabenz acetate (black column, N = 8) as compared with those after administration of physiological saline (white column, N = 8). The muscle weights of soleus muscle (Figure 12-D), plantaris muscle (Figure 12-E), and gastrocnemius muscle (Figure 12-F) of obese mice were not affected by administration of guanabenz acetate. These results indicate that 2-week administration of guanabenz to obese mice does not cause reduction of the amounts of visceral fat or muscle.

Significant difference was as follows.
Figure 1: **p < 0.01
Figure 3: *p < 0.05
Figure 4: *p < 0.05, **p < 0.01
Figure 5: *p < 0.05, **p < 0.01
Figure 6: **p < 0.01
Figure 8: **p < 0.01
Figure 9: *p < 0.05, **p < 0.01
Figure 10: **p < 0.01

### Comparison of Action Among Ameliorating Agents for Insulin Resistance

Metformin and pioglitazone are oral drugs that ameliorate insulin resistance associated with obesity-related metabolic disease (insulin dependence). Metformin primarily activates AMPK phosphorylation in the liver to ameliorate insulin resistance, and (1) exhibits hyperglycemia-mitigating action, but (2) does not exhibit anti-fatty liver action and (3) does not exhibit anti-obesity action, either. Pioglitazone dose not bind to Helz2, but binds to the functional activation domain-2 in the C-terminal side of PPARγ protein, enhancing the PPARγ transcription activity to ameliorate insulin resistance, and (1) exhibits hyperglycemia-mitigating action and (2) exhibits anti-fatty liver action in clinical situations, but there are results that administration of the ligand specific to PPARγ rather induces fatty liver (Matsusue K et al; J Clin Invest 111: 737, 2003). The growing action for fat cell (3) causes body weight gain rather than anti-obesity action. In contrast, guanabenz or a salt thereof such as an acetate salt thereof, which is identified as a low-molecular-weight compound that binds to Helz2 to lower its action, ameliorates insulin resistance, and (1) exhibits hyperglycemia-mitigating action (2) with anti-fatty liver action, (3) exhibits anti-obesity action without reduction of appetite, and (4) exhibits reducing action for high LDL cholesterol. These obesity-related metabolic diseases and the characteristics of the drug actions are summarized in Table 1 in the following.

**[Table 1]**

| | Metform in | Pioglitaz one | Guanabe nz |
|---|---|---|---|
| Insulin resistance | ↓ | ↓ | ↓ |
| Hyperglycemia | ↓ | ↓ | ↓ |
| Hypertension | → | ↓ | ↓* |
| Blood LDLc | → | → | ↓ |
| Blood HDLc | → | ↑ | → |
| Fatty liver | → | ↓ | ↓ |
| Body weight | → | ↑ | ↓ |

| | | | |
|---|---|---|---|
| ↓: lowering or mitigating action; ↑: increasing action; →: no change. * based on clinical data | | | |

The above results revealed that oral administration of guanabenz or a salt thereof such as an acetate salt thereof in a usual dose not only provides conventionally known hypotensive action through binding to central α2-adrenaline receptor, but also binds to Helz2 to lower its action and ameliorates obesity and/or insulin resistance to thereby exhibit anti-obesity action and so on without reduction of appetite, and exerts pharmacological effects for the above four different obesity-related metabolic diseases by using a single agent (drug repositioning).

Therefore, examples of disease indicated for administration of the ameliorating agent for an obesity-related metabolic disease according to the present invention, the ameliorating agent containing guanabenz or a salt of guanabenz as an active ingredient, include the following caused by obesity and/or insulin resistance.
Diabetes mellitus associated with insulin resistance
Diabetes mellitus associated with obesity
Hypertension associated with insulin resistance
Hypertension associated with obesity
Hyperlipidemia associated with insulin resistance
Hyperlipidemia associated with obesity
Fatty liver (NAFLD and NASH) associated with insulin resistance
Fatty liver (NAFLD and NASH) associated with obesity
Complication of diabetes mellitus associated with insulin resistance and all or any of hypertension, hyperlipidemia, fatty liver, coronary artery disease, and obesity disease
Complication of diabetes mellitus associated with obesity and all or any of hypertension, hyperlipidemia, fatty liver, coronary artery disease, and obesity disease
Obesity disease caused by insulin resistance
Obesity disease caused by obesity
Metabolic syndrome (consisting of increased visceral fat, abnormal glucose tolerance, mild hypertension, and mild hyperlipidemia) caused by insulin resistance
Metabolic syndrome caused by obesity
Metabolic syndrome caused by obesity

With use of formulas (content ratios) shown in Table 2, formulations are obtained in accordance with a known method described in General Rules for Preparation in The Japanese Pharmacopoeia 17th Edition.

**[Table 2]**

| Component | Formulati on Example 1 | Formulati on Example 2 | Formulati on Example 3 | Formulati on Example 4 | Formulati on Example 5 |
|---|---|---|---|---|---|
| (Dosage form) | capsule | capsule | tablet | tablet | tablet |
| Guanabenz acetate | 2.525 | 2.525 | 2.525 | 2.525 | 2.525 |
| Anhydrous lactose | 76.475 | - | - | 58.675 | - |
| D-Mannitol | - | - | 63.375 | - | - |
| Calcium hydrogen phosphate | - | 67.175 | - | - | 81.875 |
| Microcrystalli ne cellulose | - | | 30 | 25 | - |
| Corn starch | 20 | 30 | - | 10 | 10 |
| Croscarmellos e sodium | - | - | 3 | - | 5 |
| Crospovidone | - | - | - | 3 | - |
| Sodium stearyl fumarate | - | - | 1 | - | - |
| Magnesium stearate | - | 0.3 | | 0.8 | 0.3 |
| Talc | 0.5 | - | - | - | 0.3 |
| Light anhydrous silicic acid | 0.5 | - | 0.1 | - | - |
| Total | 100.0 | 100.0 | 100.0 | 100.0 | 100.0 |

## Claims

1. An ameliorating agent for an obesity-related metabolic disease comprising guanabenz or a salt of guanabenz as an active ingredient.

2. The ameliorating agent for an obesity-related metabolic disease according to claim 1, wherein the salt of guanabenz is an acetate salt.

3. The ameliorating agent for an obesity-related metabolic disease according to claim 1 or 2, wherein the obesity-related metabolic disease is caused by insulin resistance.

4. The ameliorating agent for an obesity-related metabolic disease according to any one of claims 1 to 3, wherein the obesity-related metabolic disease is caused by obesity.

5. The ameliorating agent for an obesity-related metabolic disease according to any one of claims 1 to 4, wherein the obesity-related metabolic disease is impaired glucose tolerance or diabetes mellitus.

6. The ameliorating agent for an obesity-related metabolic disease according to any one of claims 1 to 4, wherein the obesity-related metabolic disease is dyslipidemia.

7. The ameliorating agent for an obesity-related metabolic disease according to any one of claims 1 to 4, wherein the obesity-related metabolic disease is hypertension.

8. The ameliorating agent for an obesity-related metabolic disease according to any one of claims 1 to 4, wherein the obesity-related metabolic disease is hyperuricemia or gout.

9. The ameliorating agent for an obesity-related metabolic disease according to any one of claims 1 to 4, wherein the obesity-related metabolic disease is coronary artery disease.

10. The ameliorating agent for an obesity-related metabolic disease according to any one of claims 1 to 4, wherein the obesity-related metabolic disease is cerebral infarct.

11. The ameliorating agent for an obesity-related metabolic disease according to any one of claims 1 to 4, wherein the obesity-related metabolic disease is fatty liver.

12. The ameliorating agent for an obesity-related metabolic disease according to any one of claims 1 to 4, wherein the obesity-related metabolic disease is obesity-related kidney disease.

13. The ameliorating agent for an obesity-related metabolic disease according to any one of claims 1 to 12, wherein the ameliorating agent exhibits an anti-obesity action without reduction of appetite.

14. The ameliorating agent for an obesity-related metabolic disease according to any one of claims 1 to 13, wherein the obesity-related metabolic disease includes metabolic syndrome.
